# EUROPEAN PATENT APPLICATION

(11) **EP 4 418 277 A1**
(43) Date of publication of application: **21.08.2024**
(21) Application number: 22880208.8
(22) Date of filing: 09.10.2022
(51) Int. Cl.: G16H 20/30, G16H 50/00

(54) **FITNESS PLAN INFORMATION GENERATION METHOD, APPARATUS AND SYSTEM**

(30) Priority: 15.10.2021 CN 202111204326
(71) Applicant: Beijing BOE Technology Development Co., Ltd., Beijing 100176 (CN); BOE Technology Group Co., Ltd., Beijing 100015 (CN)
(72) Inventor: SU, Jing, Beijing 100176 (CN); ZHAO, Junjie, Beijing 100176 (CN); CHU, Xiao, Beijing 100176 (CN); CHEN, Shaobei, Beijing 100176 (CN)
(74) Representative: Santarelli
(86) International application number: PCT/CN2022/123934
(87) International publication number: WO 2023/061269

(57) **Abstract**

The present disclosure relates to the technical field of computers, and in particular to a fitness plan information generation method, apparatus and system. The fitness plan information generation method comprises: generating the current human body model of a user; generating a target human body model of the user according to an adjustment to the current human body model made by the user; and generating fitness plan information for the user according to the difference between the current human body model and the target human body model.

## Description

### CROSS-REFERENCE TO RELATED APPLICATIONS

The present disclosure is based on and claims priority of Chinese application for invention No. 202111204326.2, filed on Oct. 15, 2021, the disclosure of which is hereby incorporated into this disclosure by reference in its entirety.

### TECHNICAL FIELD

This disclosure relates to the field of computer technology, in particular to a generation method of fitness regimen information, a generation apparatus of fitness regimen information, a generation system of fitness regimen information and a non-transitory computer-readable storage medium.

### BACKGROUND

In related technologies, application software determines a user's current body shape and an expected body shape from some fixed types of body shape based on the user's input to recommend a fitness regimen.

### SUMMARY

According to some embodiments of the present disclosure, there is provided a generation method of fitness regimen information, comprising: generating a current human body model of a user; generating a target human body model of the user based on an adjustment of the user to the current human body model; generating the user's fitness regimen information based on differences between the current human body model and the target human body model.

In some embodiments, generating a target human body model of the user based on an adjustment of the user to the current human body model comprises: generating the target human body model of the user based on an adjustment of the user to a plurality of parts of the current human body model.

In some embodiments, the current human body model comprises a plurality of current muscle modules distributed on the current human body model, and generating a target human body model of the user based on an adjustment of the user to the current human body model comprises: generating a target human body model based on an adjustment of the user to the current muscle modules.

In some embodiments, generating a target human body model based on an adjustment of the user to the current muscle modules comprises: determining a shape change of a current muscle module based on the adjustment of the user to this current muscle module; determining a shape change of a related current muscle module of this current muscle module based on the shape change of this muscle module; generating the target human body model based on the shape change of this current muscle module and the shape change of the related current muscle module.

In some embodiments, generating the current human body model of the user comprises: generating the current human body model of the user based on current physiological feature information of the user.

In some embodiments, generating the current human body model of the user based on current physiological feature information of the user comprises: generating at least one candidate human body model based on the current physiological feature information; generating the current human body model based on an adjustment of the user to the candidate human body model.

In some embodiments, generating the current human body model based on an adjustment of the user to the candidate human body model comprises: generating the current human body model based on an adjustment of the user to a plurality of parts of the candidate human body model.

In some embodiments, generating at least one candidate human body model based on the current physiological feature information comprises: generating the candidate human body model based on the current physiological feature information using a machine learning model; the generation method further comprises: determining the current human body model as an annotation result of the current physiological feature information to generate training data; training the machine learning model using the training data.

In some embodiments, the current physiological feature information comprises first current physiological feature information which does not comprise body fat percentage (BFP) information of the user and second current physiological feature information which comprises BFP information of the user; and generating the current human body model of the user based on current physiological feature information of the user comprises: generating at least one candidate human body model based on the first current physiological feature information; generating the current human body model based on the candidate human body model and the second current physiological feature information.

In some embodiments, the candidate human body model comprises a plurality of candidate human body models, and the second current physiological feature information comprises whole BFP information of the user, wherein generating the current human body model based on the candidate human body model and the second current physiological feature information comprises: selecting the current human body model from the plurality of candidate human body models based on the whole BFP information.

In some embodiments, the second current physiological feature information comprises partial BFP information of the user, and generating the current human body model based on the candidate human body model and the second current physiological feature information comprises: adjusting a body part corresponding to the partial BFP information of the candidate human body model based on the partial BFP information to generate the current human body model.

In some embodiments, generating the current human body model of the user based on current physiological feature information of the user comprises: generating at least one candidate human body model based on the current physiological feature information; determining body length ratio information of the user based on image information of the user; adjusting the candidate human body model based on the body length ratio information to generate the current human body model.

In some embodiments, the current human body model comprises a plurality of current muscle modules distributed on the current human body model, the target human body model comprises a plurality of target muscle modules distributed on the target human body model, and generating the user's fitness regimen information based on differences between the current human body model and the target human body model comprises: generating the user's fitness regimen information based on a shape difference between a current muscle module and a corresponding target muscle module.

In some embodiments, generating the user's fitness regimen information based on differences between the current human body model and the target human body model comprises: determining target physiological feature information of the user based on the target human body model; generating the user's fitness regimen information based on differences between the current physiological feature information corresponding to the current human body model and the target physiological feature information.

In some embodiments, determining target physiological feature information of the user based on the target human body model comprises: searching for a comparable human body model which matches the target human body model in a digital human body database based on a partial shape of the target human body model; determining target physiological feature information of the user based on physiological feature information of the comparable human body model.

In some embodiments, the current physiological feature information comprises BFP information of the user, which is obtained by a body fat measurement device associated with the user.

In some embodiments, the BFP information comprises partial BFP information of the user obtained by: determining a start point and a target point on a body of the user base on a body part corresponding to the partial BFP information; determining impedance between the start point and the target point using a body fat measurement device; determining the partial BFP information based on the impedance.

In some embodiments, the generation method further comprises: determining at least one of the current physiological feature information or the current human body model as state information before training; in response to a training based on the fitness regimen information being completed by the user, determining at least one of physiological feature information after training of the user or a human body model after training as state information after training; pushing differences between the state information after training and the state information before training to the user.

In some embodiments, at least two of the current human body model, the human body model after training or a future human body model are overlapped and displayed to present the differences between at least two of the current human body model, the human body model after training or a future human body model, wherein the future human body model is predicted based on the state information before training.

In some embodiments, a change of a physiological feature of the user over time is determined based on at least two of the current physiological feature information, the physiological feature information after training or future physiological feature information, wherein the future physiological feature information is predicted based on the state information before training; a change curve is generated based on the change of the physiological feature over time to present differences between at least two of the current physiological feature information, the physiological feature information after training or future physiological feature information.

In some embodiments, at least two of the current human body model, the human body model after training, or a future human body model are displayed by overlapping with each other, wherein the future human body model is predicted based on the state information before training. the change curve and a result of the overlapping display are present to the user; in response to a selection of the user for a time point on the change curve, the current human body model, the human body model after training or the future human body model corresponding to the time point is highlighted, and/or in response to a selection of the user for the current human body model, the human body model after training or the future human body model, a time point on the change curve corresponding to the current human body model, the human body model after training or the future human body model is highlighted.

In some embodiments, the generation method further comprises: determining at least one of the current physiological feature information or the current human body model as state information before training; predicting, based on the state information before training, future state information of the user after a preset period of time, wherein the future state information comprises at least one of the future physiological feature information or the future human body model of the user; pushing the future state information to the user.

In some embodiments, predicting future state information of the user after a preset period of time based on the state information before training comprises: predicting a shape of a future muscle module of the future human body model, based on a shape of a current muscle module of the current human body model using a big data technology.

According to some other embodiments of the present disclosure, there is provided A generation apparatus of fitness regimen information, comprising: a model generation unit for generating a current human body model of a user, and generating a target human body model of the user based on an adjustment of the user to the current human body model; a regimen generation unit for generating the user's fitness regimen information based on differences between the current human body model and the target human body model.

In some embodiments, the generation apparatus further comprises: a determination unit for determining at least one of the current physiological feature information or the current human body model as state information before training, and determining, in response to a training based on the fitness regimen information being completed by the user, at least one of physiological feature information after training of the user or a human body model after training as state information after training; a push unit for pushing differences between the state information after training and the state information before training to the user.

In some embodiments, the generation apparatus further comprises: a determination unit for determining at least one of the current physiological feature information or the current human body model as state information before training; a prediction unit for predicting, based on the state information before training, future state information of the user after a preset period of time, wherein the future state information comprises the future physiological feature information or the future human body model of the user.

In some embodiments, the model generation unit generates a candidate human body model based on the current physiological feature information using a machine learning model; the generation apparatus further comprises: a training unit for determining the current human body model as an annotation result of the current physiological feature information to generate training data, and training a machine learning model using the training data.

According to still other embodiments of the present disclosure, there is provided a generation system of fitness regimen information, comprising: A generation apparatus of fitness regimen information for implementing the fitness regimen information generation method according to any one of the above embodiments; a physiological feature measurement device for obtaining current physiological features of the user.

According to still other embodiments of the present disclosure, there is provided A generation apparatus of fitness regimen information, comprising: a memory; a processor coupled to the memory, the processor configured to, based on instructions stored in the memory, carry out the fitness regimen information generation method according to any one of the above embodiments.

According to still other embodiments of the present disclosure, there is provided a non-transitory computer readable storage medium having stored thereon a computer program that, when executed by a processor, implements the fitness regimen information generation method according to any one of the above embodiments.

### BRIEF DESCRIPTION OF THE DRAWINGS

The accompanying drawings, which are incorporated in and constitute a portion of this specification, illustrate embodiments of the present disclosure and, together with the description, serve to explain the principles of the present disclosure.

The present disclosure will be more clearly understood from the following detailed description with reference to the accompanying drawings, in which:
FIG. 1 shows a flowchart of a generation method of fitness regimen information according to some embodiments of the present disclosure;
FIGS. 2a-2c show schematic diagrams of a method for generating a current human body model according to some embodiments of the present disclosure;
FIGS. 3a-3c show schematic diagrams of a method for generating a target human body model according to some embodiments of the present disclosure;
FIGS. 4a-4c show schematic diagrams of the generation method of fitness regimen information according to some embodiments of the present disclosure;
FIG. 5 shows a schematic diagram of a generation apparatus of fitness regimen information according to some embodiments of the present disclosure;
FIG. 6 shows a block diagram of the generation apparatus of fitness regimen information according to other embodiments of the present disclosure;
FIG. 7 shows a block diagram of the generation apparatus of fitness regimen information according to further embodiments of the present disclosure;
FIG. 8 shows a block diagram of a generation system of fitness regimen information according to some embodiments of the present disclosure.

### DETAILED DESCRIPTION

Various exemplary embodiments of the present disclosure will now be described in detail with reference to the accompanying drawings. Notice that, unless otherwise specified, the relative arrangement, numerical expressions and numerical values of the components and steps set forth in these examples do not limit the scope of the invention.

At the same time, it should be understood that, for ease of description, the dimensions of the plurality of parts shown in the drawings are not drawn to actual proportions.

The following description of at least one exemplary embodiment is in fact merely illustrative and is in no way intended as a limitation to the invention, its application or use.

Techniques, methods, and apparatus known to those of ordinary skill in the relevant art may not be discussed in detail, but where appropriate, these techniques, methods, and apparatuses should be considered as part of the specification.

Of all the examples shown and discussed herein, any specific value should be construed as merely illustrative and not as a limitation. Thus, other examples of exemplary embodiments may have different values.

Notice that, similar reference numerals and letters are denoted by the like in the accompanying drawings, and therefore, once an item is defined in a drawing, there is no need for further discussion in the accompanying drawings.

As mentioned above, the fitness regimen recommendations based on some fixed types of body shape results in inaccurate descriptions of users' current and target body shapes. Inaccurate descriptions which may result in inaccurate fitness regimens that lack relevancy.

In response to the above technical problems, the technical solution of the present disclosure constructs a human body model (such as a digital human body model, a three-dimensional human body model, etc.) for a specified user; With the help of the constructed human body model, the user's current body shape, post-training body shape, and expected body shape can be accurately described.

This allows personalized processing for different users to improve the relevance and accuracy of fitness regimens. In this way, precise training effects can be achieved and the enthusiasm for training and the user experience can be improved. For example, the technical solution of the present disclosure can be realized through the following embodiments.

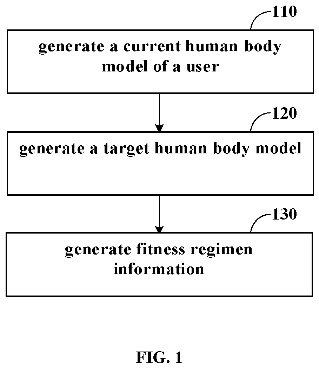

FIG. 1 shows a flowchart of a generation method of fitness regimen information according to some embodiments of the present disclosure.

As shown in FIG. 1, in step 110, a current human body model of a user is generated. For example, a current human body model can be generated for a user based on current physiological feature information of the user. The current physiological feature information of the user obtained by the system may comprise gender, age, height, weight, body fat percentage, etc.

In some embodiments, the current physiological feature information may be obtained by requesting input from the user or from a user-associated device. For example, the user-associated device may be a user-associated body fat measurement device (such as a body fat scale) that can provide current physiological feature information such as body weight and body fat percentage.

In some embodiments, based on the obtained current physiological feature information, a three-dimensional human body model is generated as the user's current human body model.

In some embodiments, the current physiological feature information comprises first current physiological feature information and second current physiological feature information. The first current physiological feature information does not comprise BFP information of the user, while the second current physiological feature information comprises BFP information of the user. At least one candidate human body model is generated based on the first current physiological feature information. The current human body model is generated based on the at least one candidate human body model and the second current physiological feature information.

For example, the system generates at least one candidate human body model based on the first current physiological feature information such as height, weight, and BWH; the system adjusts the at least one candidate human body model based on the second current physiological feature information such as BFP. The user can adjust a 3D human body model by dragging its adjustment curve to generate a current human body model which matches the user's current body shape.

In some embodiments, at least one candidate human body model is generated based on the current physiological feature information using a machine learning model; the current human body model is determined as an annotation result of the current physiological feature information to generate training data; and the machine learning model is trained using the training data.

In this way, the current human body model adjusted by the user can be used as training data for the machine learning model, and a more accurate current human body model can be generated by expanding the training samples and continuously optimizing the machine learning model.

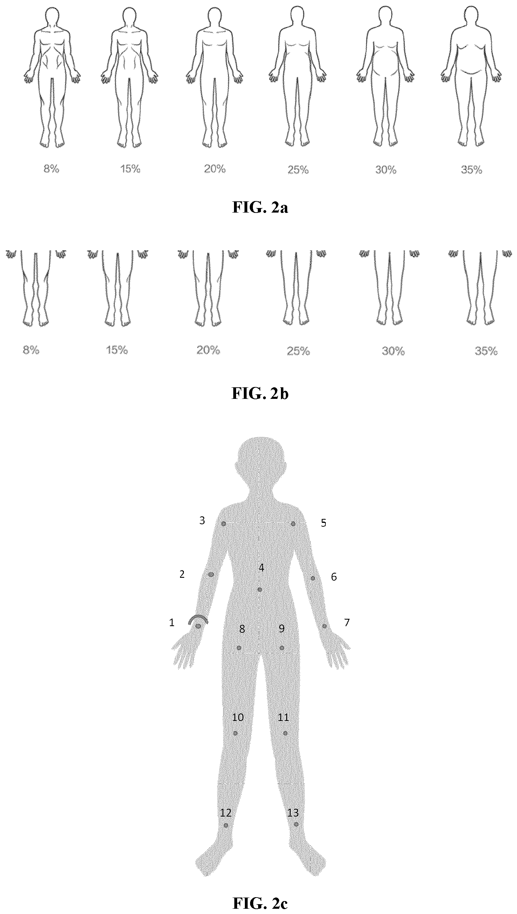

FIGS. 2a-2b show schematic diagrams of a method for generating a current human body model according to some embodiments of the present disclosure.

As shown in FIG. 2a, in some embodiments, the at least one candidate human body model comprises a plurality of candidate human body models, and the second current physiological feature information comprises whole BFP information of the user. A current human body model is selected from the plurality of candidate human body models based on the whole BFP information.

For example, the whole BFP information can be directly measured and obtained through a device such as a body fat scale, or directly obtained from a medical examination report. That is to say, the whole BFP information can be easily obtained, and it is more convenient to build a human body model based on the whole BFP information.

For example, a plurality of candidate human body models can be generated based on the first current physiological feature information such as height and weight. Because the volume of fat is three times that of muscle for the same weight, the lower the BFP, the thinner the user will be. Therefore, a body shape of the user cannot be determined solely based on the first physiological feature information, such as height and weight.

When the BFP obtained by the system is whole BFP information, a current human body model which matches the whole BFP information is selected from the candidate human body models based on the whole BFP information, that is, a candidate human body model that best matches the whole BFP information among the candidate human body models. For example, if the user has a BFP of 20%, a candidate human body model with a BFP of 20% will be selected as the current human body model. FIG. 2a shows current human body models with different BFP values.

In some embodiments, the system can also directly generate a current human body model based on the first current physiological feature information such as height and weight entered by the user, and the second current physiological feature information such as whole BFP.

As shown in FIG. 2b, in some embodiments, the second current physiological feature information comprises partial BFP information of the user. A body part corresponding to the partial BFP information of the candidate human body model is adjusted based on the partial BFP information to generate the current human body model.

The human body model created based on partial BFP information can more accurately describe the user's current body shape at the level of body part granularity, thereby improving the relevancy and accuracy of the generated fitness regimen information.

For example, the BFP obtained by the system is partial BFPs of a plurality of parts of the human body. For example, the partial BFPs comprise an upper body BFP, and the upper body structure of the candidate human body model can be adjusted based on the upper body BFP; The partial BFPs comprise a lower body BFP that can be used to adjust the lower body structure. FIG. 2b shows current human body models with different lower body BFPs.

In some embodiments, the current physiological feature information comprises BFP information of the user, which is obtained by a body fat measurement device associated with the user.

For example, the BFP information comprises partial BFP information of the user that is obtained by: determining a start point and a target point on a body of the user base on a body part corresponding to the partial BFP information; determining impedance between the start point and the target point using a body fat measurement device; determining the partial BFP information based on the impedance.

FIG. 2c shows a schematic diagram of a method for generating a current human body model according to some embodiments of the present disclosure.

As shown in FIG. 2c, partial BFP information such as left upper limb fat, left skeletal muscle, right upper limb fat, right skeletal muscle, and abdominal fat is obtained using a body fat measurement device; and a body part corresponding to the partial BFP information of the human body model is adjusted based on the partial BFP information.

In some embodiments, the impedance between a start point and a target point can be measured, and the BFP of a part between the start point and the target point can be calculated based on the impedance, thereby measuring the BFP of each body part.

For example, an external device can be used to measure the BFPs of a plurality of parts of a body of the user. The external device may be a wearable device with a body fat measuring function.

For example, measurement point 1 is the start point, measurement point 2 is the target point, and the part between the start point and the target point corresponds to the right forearm of the human body; measurement point 2 is the start point, measurement point 3 is the target point, and the part between the start point and the target point corresponds to the right upper arm of the human body; measurement point 1 is the start point, measurement point 3 is the target point, and the part between the start point and the target point corresponds to the right arm of the human body.

In some embodiments, a candidate human body model is generated based on the current physiological feature information; the candidate human body model is adjusted by the user to generate the current human body model. For example, the user can fine tune the generated human body model to generate a current human body model which matches the user's current body shape.

Body length ratio is an important factor in the construction of a digital or three-dimensional human body model. For example, some people have longer upper bodies and shorter lower bodies, or shorter upper bodies and longer lower bodies. Therefore, the body length ratio factor can be considered to generate a digital or 3D human body model as the current human body model.

In some embodiments, a candidate human body model is generated based on the current physiological feature information; body length ratio information of the user is determined based on image information of the user; and the candidate human body model is adjusted based on the body length ratio information to generate the current human body model.

For example, physiological feature information such as the user's height, weight, chest circumference, waist circumference and flat or partial photos of the user are obtained. A photo of a part that the user wishes to strengthen is obtained as a basis for generating the current human body model.

In some embodiments, the system can obtain image information of the user, such as full or half body photos, videos, etc. The users' photos and videos can be analyzed to calculate the proportions of the head, upper body, and lower body of the human body. Based on the calculation result, proportion adjustment is performed on the candidate human body model to generate the current human body model, so that the current human body model can match a body of the user's body length ratio. For example, if the upper part of the human body is long, the waist of the body model will be long.

For example, the navel is used as a boundary between the upper and lower body. If Full body length : Lower body length = Lower body length : upper body length = 1:1.618 is satisfied, the body length ratio of the human body conforms to the golden ratio.

In step 120, a target human body model is generated for the user based on an adjustment of the user to the current human body model. For example, the target human body model is generated for the user based on an adjustment of the user to a plurality of parts of the current human body model.

For example, the parts may be the chest, the abdomen, the arms, the thighs, the calves, etc.; the user adjustments may comprise shape adjustments to these parts, such as adjusting the size of these parts by dragging operation or clicking on adjustment buttons; the user adjustments may also comprise adjustments to subcutaneous tissues such as muscle and fat in these areas.

In some embodiments, a target human body model is generated for the user based on the user's dragging operations on the current human body model or clicks on adjustment buttons.

For example, the user can perform operates on the current human body model to change the current human body model, and generate the target human body model. The operation can be as simple as clicking a plus or minus button to adjust any part of the current human body model; it may also be a direct drag and drop on any part of the current human body model to expand or shrink that part.

The system can provide a plurality of recommended human body models, and the user can select a target human body model from the recommended human body models. The user can make further adjustments to the selected recommended human body model to generate the target human body model.

The user first selects needs such as the body parts they want to focus on or the effects they want to achieve. For example, the selection can be performed via a menu. Then the system generates a recommended human body model based on the user's needs.

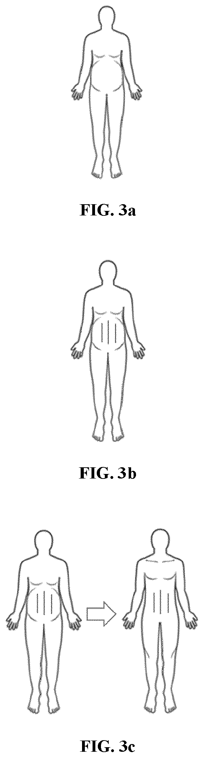

FIGS. 3a-3b show schematic diagrams of a method for generating a target human body model according to some embodiments of the present disclosure.

FIG. 3a shows the current human body model of a user. As shown in FIG. 2b, an adjustment to the abdomen is made on the current human body model by, for example, drawing three abdominal lines, to generate the user's target human body model.

In some embodiments, the current human body model comprises a plurality of current muscle modules distributed on the current human body model. A target human body model is generated based on an adjustment of the user to the current muscle modules.

For example, the current human body model is a digital 3D human body model that can show the distribution of muscles over the entire body. The user can adjust the shape of each of the current muscle modules to generate the shape of a target muscle model, thereby generating the target human body model.

In some embodiments, a shape change of any current muscle module is determined based on the adjustment of the user to the current muscle module; Shape changes of related current muscle modules of the current muscle module are determined based on the shape change of the current muscle module; the target human body model is generated based on the shape change of the current muscle module and the shape change of the related current muscle module.

For example, a muscle module on a human body model can correspond to a body part where the muscle module is located; an adjustment to a muscle module may comprise adjustments to subcutaneous tissues such as muscle and fat on a corresponding part of the human body.

This allows the user to accurately describe the desired fitness goal without the need for professional knowledge of human muscle or fitness. This fitness goal can comprise the specific target shape of target training parts, thereby improving the accuracy of the fitness regimen information.

FIG. 3c show a schematic diagram of a method for generating a target human body model according to some embodiments of the present disclosure.

As shown in FIG. 3c, some adaptive modifications can be made because the effect of training may comprise changes in some surrounding body parts of a body part, rather than only in the body part. For example, if the user has drawn three abdominal lines on the abdomen of the current human body model, adjustments can be made to related parts such as the chest, legs, and arms based on the adjustment to the abdomen.

In step 130, fitness regimen information is generated for the user based on differences between the current human body model and the target human body model.

In some embodiments, the current human body model comprises a plurality of current muscle modules distributed on the current human body model, and the target human body model comprises a plurality of target muscle modules distributed on the target human body model. Fitness regimen information is generated for the user based on a shape difference between a current muscle module and a corresponding target muscle module.

In this way, different and more accurate fitness regimen information can be generated based on the target muscle shape that the user wants to achieve. For example, to increase chest circumference by 10cm, different fitness regimen information can be generated for different chest muscle shapes according to the target muscle model. Thus, targeted fitness regimens can be generated based on the user's target muscle shape.

In some embodiments, target physiological feature information of the user is determined based on the target human body model; fitness regimen information is generated for the user based on differences between the current physiological feature information and the target physiological feature information.

For example, a comparable human body model matching the target human body model can be searched in a digital human body database based on a partial shape of the target human body model; and target physiological feature information of the user is determined based on physiological feature information of the comparable human body model.

For example, based on the differences between the current human body model and a target human body shape, body parts which the user want to work out and target improvement values can be obtained, and then recommended fitness regimen information can be generated.

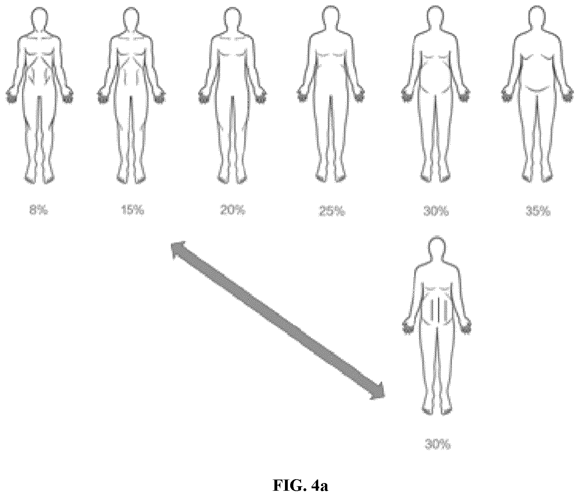

FIG. 4a shows a schematic diagram of the generation method of fitness regimen information according to some embodiments of the present disclosure.

As shown in FIG. 4a, the user's current BFP is 30%, and the expected target human body model matches the abdomen part of a candidate human body model with a BFP of 15%. It may be determined that the user needs to reduce the abdominal BFP from 30% to 150 or less; fitness regimen information is then generated with the goal of reducing BFP by 15%.

In some embodiments, after the user trains for a period of time based on the fitness regimen information, the system can generate a digital human body model or 3D model of the user as a human body model after training.

For example, step 110 can be repeated, and the system judges the effectiveness of the training based on the initially generated pre-training human body model (i.e. the current human body model) and the human body model after training after a period of training.

For example, the system can generate new fitness regimen information based on the post-training model after a period of training and a newly generated target human body model.

In some embodiments, at least one of the current physiological feature information or the current human body model is determined as state information before training; in response to a training based on the fitness regimen information being completed by the user, at least one of physiological feature information after training of the user or a human body model after training is determined as state information after training; and differences between the state information after training and the state information before training are pushed to the user.

For example, at least two of the current human body model, the human body model after training or a future human body model are overlapped and displayed to present the differences between at least two of the current human body model, the human body model after training or the future human body model, wherein the future human body model is predicted based on the state information before training.

For example, a change of a physiological feature of the user over time is determined based on at least two of the current physiological feature information, the physiological feature information after training or future physiological feature information, wherein the future physiological feature information is predicted based on the state information before training; a change curve is generated based on the change of the physiological feature over time to present differences between at least two of the current physiological feature information, the physiological feature information after training or the future physiological feature information.

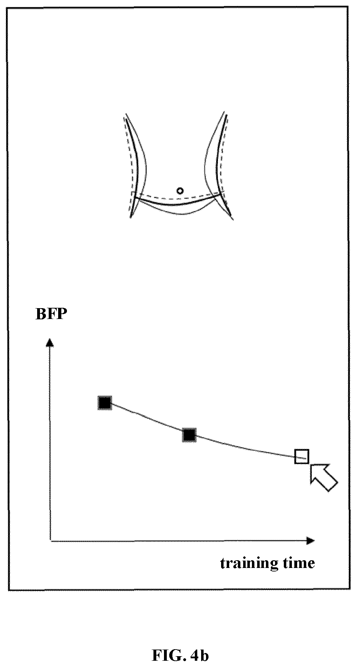

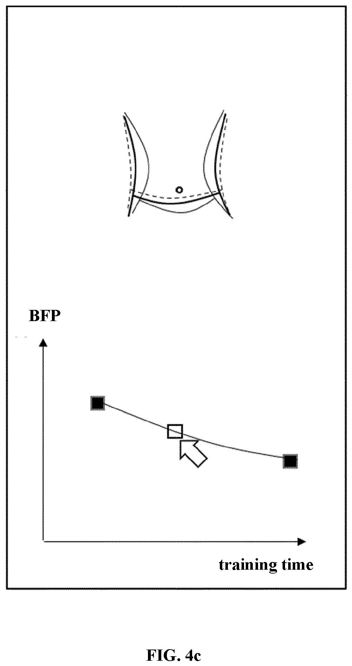

FIGS. 4b and 4c show schematic diagrams of the generation method of fitness regimen information according to some embodiments of the present disclosure.

As shown in FIGS. 4b and 4c, at least two of the current human body model, the human body model after training, or a future human body model are displayed by overlapping with each other, wherein the future human body model is predicted based on the state information before training; and a change curve and an overlapping display result are present to the user.

For example, in FIGS. 4b and 4c, the human body model denoted by the dotted lines is the current human body model, the human body model denoted by the thick lines is the post-training human body model, and the human body model denoted by the thin lines is the future human body model, corresponding to three time points on the change curve below, respectively.

In response to a selection of the user for a time point on the change curve, the current human body model, the human body model after training or the future human body model corresponding to the time point is highlighted, and/or in response to a selection of the user for the current human body model, the human body model after training or the future human body model, a time point on the change curve corresponding to the current human body model, the human body model after training or the future human body model is highlighted.

For example, in FIG. 4b, the user moves the mouse to the third time point on the change curve, the future human body model corresponding to that time point is then highlighted. In FIG. 4c, the user moves the mouse to the second time point on the change curve, the human body model after training corresponding to that time point is highlighted.

For example, when the user moves the mouse to a body model, a time point corresponding to that body model in the change curve below is highlighted.

In this way, the user can be presented with comparative results before and after training, making the experience more fun and interactive, and increasing the user's confidence in fitness training.

In some embodiments, at least one of the current physiological feature information or the current human body model is determined as state information before training. Future state information of the user after a preset period of time is predicted based on the state information before training. The future state information comprises at least one of the future physiological feature information or the future human body model of the user. The future state information is then pushed to the user.

For example, the current human body model, the human body model after training and the future human body model are overlapped and displayed to present differences between the current human body model, the human body model after training and the future human body model.

For example, a shape of a future muscle module of the future human body model is predicted, based on a shape of a current muscle module of the current human body model using a big data technology.

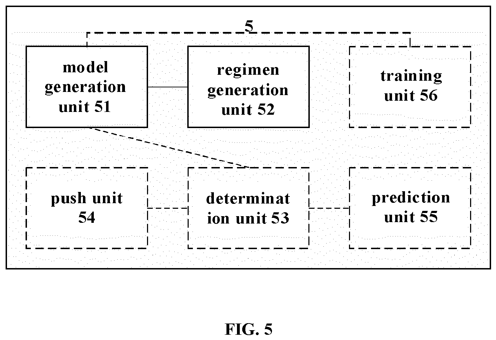

FIG. 5 shows a schematic diagram of A generation apparatus of fitness regimen information according to some embodiments of the present disclosure.

As shown in FIG. 5, the apparatus 5 for generating the fitness regimen information comprises a model generation unit 51 and a regimen generation unit 52.

The model generation unit 51 is used for generating a current human body model of a user, and generating a target human body model of the user based on an adjustment of the user to the current human body model. For example, the model generation unit 51 generates a current human body model for a user based on current physiological feature information of the user.

The regimen generation unit 52 generates the user's fitness regimen information based on differences between the current human body model and the target human body model.

In some embodiments, the generation apparatus 5 further comprises a determination unit 53 for determining at least one of the current physiological feature information or the current human body model as state information before training; determining, in response to a training based on the fitness regimen information being completed by the user, at least one of physiological feature information after training of the user or a human body model after training as state information after training.

The generation apparatus 5 further comprises a push unit 54 for pushing differences between the state information after training and the state information before training to the user.

In some embodiments, the determination unit 53 determines at least one of the current physiological feature information or the current human body model as state information before training. The generation apparatus 5 further comprises a prediction unit 55 for predicting, based on the state information before training, future state information of the user after a preset period of time, wherein the future state information comprises the future physiological feature information or the future human body model of the user.

In some embodiments, the current physiological feature information comprises first current physiological feature information which does not comprise body fat percentage (BFP) information of the user and second current physiological feature information which comprises BFP information of the user. The model generation unit 51 is used for generating at least one candidate human body model based on the first current physiological feature information; generating the current human body model based on the candidate human body model and the second current physiological feature information.

In some embodiments, the candidate human body model comprises a plurality of candidate human body models, and the second current physiological feature information comprises whole BFP information of the user. In some embodiments, the current human body model is selected from the candidate human body models based on the whole BFP information.

In some embodiments, the second current physiological feature information is partial BFP information of the user. The model generation unit 51 is used for adjusting a body part corresponding to the partial BFP information of the candidate human body model based on the partial BFP information to generate the current human body model.

The model generation unit 51 is used for generating at least one candidate human body model based on the current physiological feature information; generating the current human body model based on an adjustment of the user to the candidate human body model.

The model generation unit 51 is used for generating at least one candidate human body model based on the current physiological feature information; determining body length ratio information of the user based on image information of the user; and adjusting the candidate human body model based on the body length ratio information to generate the current human body model.

In some embodiments, the current human body model comprises a plurality of current muscle modules distributed on the current human body model. The model generation unit 51 is used for generating a target human body model based on an adjustment of the user to the current muscle modules.

The model generation unit 51 is used for determining a shape change of a current muscle module based on the adjustment of the user to the current muscle module; determining a shape change of a related current muscle module of the current muscle module based on the shape change of the current muscle module; and generating the target human body model based on the shape change of the current muscle module and the shape change of the related current muscle module.

In some embodiments, the model generation unit 51 is used for generating the target human body model of the user based on the user's drag-and-drop operations on the current human body model or clicks on adjustment buttons.

In some embodiments, the regimen generation unit 52 is used for generating the fitness regimen information for the user based on a shape difference between a current muscle module and a corresponding target muscle module. The current human body model comprises a plurality of current muscle modules distributed on the current human body model, and the target human body model comprises a plurality of target muscle modules distributed on the target human body model.

In some embodiments, the regimen generation unit 52 is used for determining target physiological feature information of the user based on the target human body model; generating the fitness regimen information for the user based on differences between the current physiological feature information and the target physiological feature information.

In some embodiments, the regimen generation unit 52 is used for searching a digital human body database for a comparable human body model which matches the target human body model based on a partial shape of the target human body model; determining target physiological feature information of the user based on physiological feature information of the comparable human body model.

In some embodiments, the current physiological feature information comprises BFP information of the user, which is obtained by a body fat measurement device associated with the user.

In some embodiments, the BFP information comprises partial BFP information of the user obtained by: determining a start point and a target point on a body of the user base on a body part corresponding to the partial BFP information; determining impedance between the start point and the target point using a body fat measurement device; determining the partial BFP information based on the impedance.

In some embodiments, the determination unit 53 is used for determining a change of a physiological feature of the user over time based on the current physiological feature information and the physiological feature information after training; the push unit 54 is used for generating a change curve based on the change of the physiological feature over time to present differences between the current physiological feature information and the physiological feature information after training.

In some embodiments, the determination unit 53 is used for determining at least one of the current physiological feature information or the current human body model as state information before training; the prediction unit 55 is used for predicting, based on the state information before training, future state information of the user after a preset period of time, wherein the future state information comprises at least one of future physiological feature information or a future human body model of the user; the push unit 54 is used for pushing the future state information to the user.

The prediction unit 55 is used for predicting a shape of a future muscle module of the future human body model, based on a shape of a current muscle module of the current human body model using a big data technology.

In some embodiments, the model generation unit 51 is used for generating at least one candidate human body model based on the current physiological feature information using a machine learning model. The generation apparatus 5 further comprises a training unit 56 for determining the current human body model as an annotation result of the current physiological feature information to generate training data, and training a machine learning model using the training data.

In some embodiments, the generation apparatus 5 further comprises a push unit 54. The push unit 54 is used for displaying at least two of the current human body model, the human body model after training or the future human body model overlappingly to present differences between at least two of the current human body model, the human body model after training or the future human body model, wherein the future human body model is predicted based on the state information before training.

In some embodiments, the push unit 54 is used for determining a change of a physiological feature of the user over time based on at least two of the current physiological feature information, the physiological feature information after training or future physiological feature information, wherein the future physiological feature information is predicted based on the state information before training; generating a change curve based on the change of the physiological feature over time to present differences between at least two of the current physiological feature information, the physiological feature information after training or future physiological feature information.

In some embodiments, the push unit 54 is used for displaying at least two of the current human body model, the human body model after training, or a future human body model overlappingly, wherein the future human body model is predicted based on the state information before training; presenting the change curve and a result of the overlapping display to the user; in response to a selection of the user for a time point on the change curve, highlighting the current human body model, the human body model after training or the future human body model corresponding to the time point, and/or in response to a selection of the user for the current human body model, the human body model after training or the future human body model, highlighting a time point on the change curve corresponding to the current human body model, the human body model after training or the future human body model.

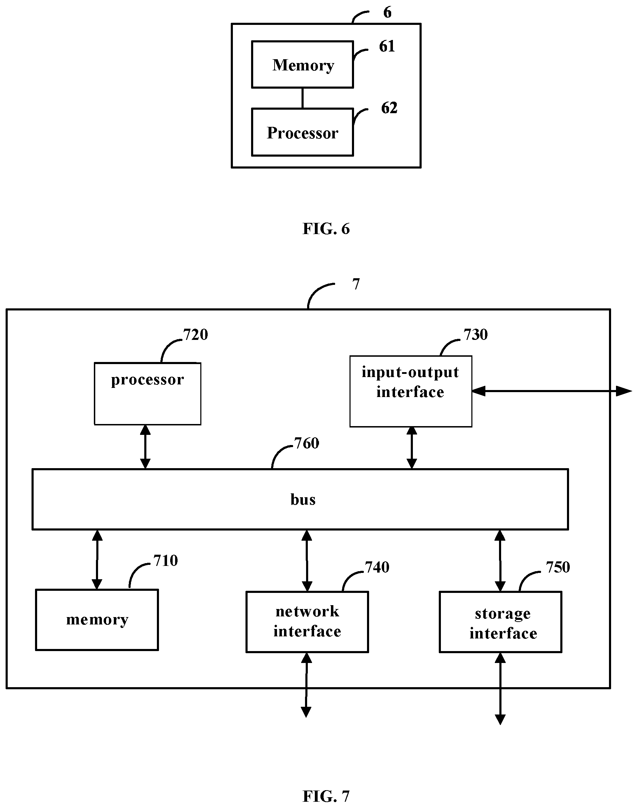

FIG. 6 shows a block diagram of the generation apparatus of fitness regimen information according to other embodiments of the present disclosure.

As shown in FIG. 6, the apparatus 6 for generating the fitness regimen information of this embodiment comprises: a memory 61 and a processor 62 coupled to the memory 61, the processor 62 configured to, based on instructions stored in the memory 61, carry out the generation method of fitness regimen information according to any one of the embodiments of the present disclosure.

Wherein, the memory 51 may comprise, for example, system memory, a fixed non-transitory storage medium, or the like. The system memory stores, for example, an operating system, applications, a boot loader, a database, and other programs.

FIG. 7 shows a block diagram of the generation apparatus of fitness regimen information according to further embodiments of the present disclosure.

As shown in FIG. 7, the apparatus 7 for generating the fitness regimen information of this embodiment comprises: a memory 710 and a processor 720 coupled to the memory 710, the processor 720 configured to, based on instructions stored in the memory 710, carry out the generation method of fitness regimen information according to any one of the embodiments of the present disclosure.

The memory 710 may comprise, for example, system memory, a fixed non-transitory storage medium, or the like. The system memory stores, for example, an operating system, application programs, a boot loader, and other programs.

The apparatus 7 for generating the fitness regimen information may further comprise an input-output interface 730, a network interface 740, a storage interface 750, and the like. These interfaces 730, 740, 750, the memory 710 and the processor 720 may be connected through a bus 760, for example. Wherein, the input-output interface 730 provides a connection interface for input-output devices such as a display, a mouse, a keyboard, a touch screen, a microphone, a loudspeaker, etc. The network interface 740 provides a connection interface for various networked devices. The storage interface 750 provides a connection interface for external storage devices such as an SD card and a USB flash disk.

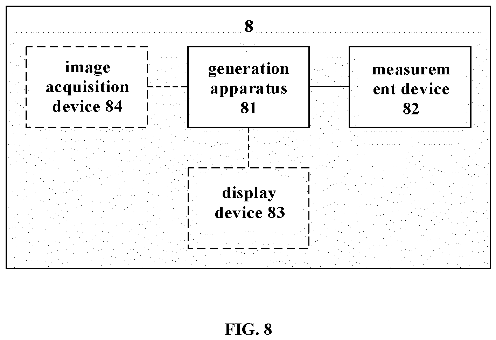

FIG. 8 shows a block diagram of a generation system of fitness regimen information according to some embodiments of the present disclosure.

As shown in FIG. 8, the system 8 for generating the fitness regimen information comprises: an apparatus 81 for generating the fitness regimen information according to any one of the above embodiments, the apparatus 81 implementing the generation method of fitness regimen information according to any one of the above embodiments; a physiological feature measurement device 82 for obtaining current physiological features of the user.

In some embodiments, the physiological feature measurement device 82 comprises at least one of a weight measurement device, a height measurement device, or a body fat measurement device.

In some embodiments, the generation system 8 further comprises a display device for displaying the user's current and target human body models.

In some embodiments, the generation system 8 further comprises an image acquisition device 84 for acquiring image information of the user.

Those skilled in the art should understand that the embodiments of the present disclosure may be provided as a method, a system, or a computer program product. Therefore, embodiments of the present disclosure can take the form of an entirely hardware embodiment, an entirely software embodiment or an embodiment containing both hardware and software elements. Moreover, the present disclosure may take the form of a computer program product embodied on one or more computer-usable non-transitory storage media (comprising but not limited to disk storage, CD-ROM, optical memory, etc.) having computer-usable program code embodied therein.

Heretofore, the method, apparatus and generation system of fitness regimen information and the non-transitory computer-readable storage medium according to the present disclosure have been described in detail. In order to avoid obscuring the concepts of the present disclosure, some details known in the art are not described. Based on the above description, those skilled in the art can understand how to implement the technical solutions disclosed herein.

The method and system of the present disclosure may be implemented in many ways. For example, the method and system of the present disclosure may be implemented by software, hardware, firmware, or any combination of software, hardware, and firmware. The above sequence of steps of the method is merely for the purpose of illustration, and the steps of the method of the present disclosure are not limited to the above-described specific order unless otherwise specified. In addition, in some embodiments, the present disclosure may also be implemented as programs recorded in a recording medium, which comprise machine-readable instructions for implementing the method according to the present disclosure. Thus, the present disclosure also covers a recording medium storing programs for executing the method according to the present disclosure.
Although some specific embodiments of the present disclosure have been described in detail by way of example, those skilled in the art should understand that the above examples are only for the purpose of illustration and are not intended to limit the scope of the present disclosure. It should be understood by those skilled in the art that the above embodiments may be modified without departing from the scope and spirit of the present disclosure. The scope of the disclosure is defined by the following claims.

## Claims

1. A generation method of fitness regimen information, comprising:
generating a current human body model of a user;
generating a target human body model of the user based on an adjustment of the user to the current human body model; and
generating the fitness regimen information for the user based on differences between the current human body model and the target human body model.

2. The generation method according to claim 1, wherein the generating the target human body model of the user based on the adjustment of the user to the current human body model comprises:
generating the target human body model of the user based on the adjustment of the user to a plurality of parts of the current human body model.

3. The generation method according to claim 2, wherein the current human body model comprises a plurality of current muscle modules distributed on the current human body model;
the generating the target human body model of the user based on the adjustment of the user to the plurality of parts of the current human body model comprises:
generating the target human body model based on the adjustment of the user to the plurality of current muscle modules.

4. The generation method according to claim 3, wherein the generating the target human body model based on the adjustment of the user to the plurality of current muscle modules comprises:
determining a shape change of a current muscle module based on the adjustment of the user to the current muscle module;
determining a shape change of a related current muscle module of the current muscle module based on the shape change of the current muscle module; and
generating the target human body model based on the shape change of the current muscle module and the shape change of the related current muscle module.

5. The generation method according to any one of claims 1 to 4, wherein the generating the current human body model of the user comprises:
generating the current human body model of the user based on current physiological feature information of the user.

6. The generation method according to claim 5, wherein the generating the current human body model of the user based on the current physiological feature information of the user comprises:
generating at least one candidate human body model based on the current physiological feature information; and
generating the current human body model based on an adjustment of the user to the candidate human body model.

7. The generation method according to claim 6, wherein the generating the current human body model based on the adjustment of the user to the candidate human body model comprises:
generating the current human body model based on the adjustment of the user to a plurality of parts of the candidate human body model.

8. The generation method according to claim 6, wherein the generating the candidate human body model based on the current physiological feature information comprises:
generating the candidate human body model based on the current physiological feature information using a machine learning model;
the generation method further comprises:
determining the current human body model as an annotation result of the current physiological feature information to generate training data; and
training the machine learning model using the training data.

9. The generation method according to claim 5, wherein the current physiological feature information comprises first current physiological feature information which does not comprise body fat percentage (BFP) information of the user and second current physiological feature information which comprises the BFP information of the user,
the generating the current human body model of the user based on the current physiological feature information of the user comprises:
generating at least one candidate human body model based on the first current physiological feature information; and
generating the current human body model based on the candidate human body model and the second current physiological feature information.

10. The generation method according to claim 9, wherein the candidate human body model comprises a plurality of candidate human body models, and the second current physiological feature information comprises whole BFP information of the user,
the generating the current human body model based on the candidate human body model and the second current physiological feature information comprises:
selecting the current human body model from the plurality of candidate human body models based on the whole BFP information.

11. The generation method according to claim 9, wherein the second current physiological feature information comprises partial BFP information of the user,
the generating the current human body model based on the at least one candidate human body model and the second current physiological feature information comprises:
adjusting a body part corresponding to the partial BFP information of the least one candidate human body model based on the partial BFP information to generate the current human body model.

12. The generation method according to claim 5, wherein the generating the current human body model of the user based on the current physiological feature information of the user comprises:
generating at least one candidate human body model based on the current physiological feature information;
determining body length ratio information of the user based on image information of the user; and
adjusting the at least one candidate human body model based on the body length ratio information to generate the current human body model.

13. The generation method according to any one of claims 1 to 4, wherein the current human body model comprises a plurality of current muscle modules distributed on the current human body model, and the target human body model comprises a plurality of target muscle modules distributed on the target human body model,
the generating the fitness regimen information for the user based on the differences between the current human body model and the target human body model comprises:
generating the fitness regimen information for the user based on a shape difference between a current muscle module and a corresponding target muscle module.

14. The generation method according to any one of claims 1 to 4, wherein the generating the fitness regimen information for the user based on the differences between the current human body model and the target human body model comprises:
determining target physiological feature information of the user based on the target human body model; and
generating the fitness regimen information for the user based on differences between current physiological feature information corresponding to the current human body model and the target physiological feature information.

15. The generation method according to claim 14, wherein the determining the target physiological feature information of the user based on the target human body model comprises:
searching for a comparable human body model which matches the target human body model in a digital human body database based on a partial shape of the target human body model; and
determining the target physiological feature information of the user based on physiological feature information of the comparable human body model.

16. The generation method according to claim 5, wherein the current physiological feature information comprises BFP information of the user, which is obtained by a body fat measurement device associated with the user.

17. The generation method according to claim 16, wherein the BFP information comprises partial BFP information of the user obtained by:
determining a start point and a target point on a body of the user base on a body part corresponding to the partial BFP information;
determining impedance between the start point and the target point using the body fat measurement device; and
determining the partial BFP information based on the impedance.

18. The generation method according to claim 5, further comprising:
determining at least one of the current physiological feature information or the current human body model as state information before training;
determining, in response to a training based on the fitness regimen information being completed by the user, at least one of physiological feature information after training of the user or a human body model after training as state information after training; and
pushing differences between the state information after training and the state information before training to the user.

19. The generation method according to claim 18, further comprising:
displaying at least two of the current human body model, the human body model after training or a future human body model overlappingly to present differences between at least two of the current human body model, the human body model after training or the future human body model, wherein the future human body model is predicted based on the state information before training.

20. The generation method according to claim 18, further comprising:
determining a change of a physiological feature of the user over time based on at least two of the current physiological feature information, the physiological feature information after training or future physiological feature information, wherein the future physiological feature information is predicted based on the state information before training; and
generating a change curve based on the change of the physiological feature over time to present the differences between at least two of the current physiological feature information, the physiological feature information after training or future physiological feature information.

21. The generation method according to claim 20, further comprising:
displaying at least two of the current human body model, the human body model after training, or a future human body model overlappingly, wherein the future human body model is predicted based on the state information before training;
presenting the change curve and a result of the overlapping display to the user; and
highlighting the current human body model, the human body model after training or the future human body model corresponding to a time point, in response to a selection of the user for the time point on the change curve, and/or highlighting a time point on the change curve corresponding to the current human body model, the human body model after training or the future human body model, in response to a selection of the user for the current human body model, the human body model after training or the future human body model.

22. The generation method according to claim 5, further comprising:
determining at least one of the current physiological feature information or the current human body model as state information before training;
predicting, based on the state information before training, future state information of the user after a preset period of time, wherein the future state information comprises at least one of the future physiological feature information or the future human body model of the user; and
pushing the future state information to the user.

23. The generation method according to claim 22, wherein the predicting, based on the state information before training, future state information of the user after the preset period of time comprises:
predicting a shape of a future muscle module of the future human body model, based on a shape of a current muscle module of the current human body model using a big data technology.

24. A generation apparatus of fitness regimen information, comprising:
a model generation unit for generating a current human body model of a user, and generating a target human body model of the user based on an adjustment of the user to the current human body model; and
a regimen generation unit for generating the fitness regimen information for the user based on differences between the current human body model and the target human body model.

25. The generation apparatus according to claim 24, further comprising:
a determination unit for determining at least one of the current physiological feature information or the current human body model as state information before training, and determining, in response to a training based on the fitness regimen information being completed by the user, at least one of physiological feature information after training of the user or a human body model after training as state information after training; and
a push unit for pushing differences between the state information after training and the state information before training to the user.

26. The generation apparatus according to claim 24, further comprising:
a determination unit for determining the current physiological feature information or the current human body model as state information before training; and
a prediction unit for predicting, based on the state information before training, future state information of the user after a preset period of time, wherein the future state information comprises the future physiological feature information or the future human body model of the user.

27. The generation apparatus according to claim 24, wherein the model generation unit is used for generating the at least one candidate human body model based on the current physiological feature information using a machine learning model;
the generation apparatus further comprises:
a training unit for determining the current human body model as an annotation result of the current physiological feature information to generate training data, and training a machine learning model using the training data.

28. A generation apparatus of fitness regimen information, comprising:
a memory; and
a processor coupled to the memory, the processor configured to, based on instructions stored in the memory, carry out a generation method of fitness regimen information according to any one of claims 1 to 23.

29. A generation system of fitness regimen information, comprising:
a generation apparatus of fitness regimen information according to any one of claims 24 to 28;
a physiological feature measurement device for obtaining a current physiological feature of a user.

30. The generation system according to claim 29, wherein the physiological feature measurement device comprises at least one of a weight measurement device, a height measurement device, or a body fat measurement device.

31. The generation system according to claim 29, further comprising:
a display device for displaying a current human body model and a target human body model of the user.

32. The generation system according to claim 29, further comprising:
an image acquisition device for acquiring image information of the user.

33. A non-transitory computer-readable storage medium stored thereon a computer program that, when executed by a processor, implements a generation method of fitness regimen information according to any one of claims 1 to 23.
